# EUROPEAN PATENT SPECIFICATION

(11) **EP 4 311 504 B1**
(45) Date of publication and mention of the grant of the patent: **02.10.2024**
(21) Application number: 23180237.2
(22) Date of filing: 20.06.2023
(51) Int. Cl.: A61B 17/15, A61B 17/84, A61B 17/17

(54) **SAW BLADE GUIDE FOR DOUBLE AND TRIPLE PELVIC OSTEOTOMIES IN DOGS**
SÄGEBLATTFÜHRUNG FÜR DOPPEL- UND DREIFACHBECKENOSTEOTOMIEN BEI HUNDEN
GUIDE DE LAME DE SCIE POUR OSTÉOTOMIES PELVIENNES DOUBLES ET TRIPLES CHEZ LES CHIENS

(30) Priority: 30.07.2022 PL 44188622
(43) Date of publication of application: 31.01.2024
(73) Proprietor: Uniwersytet Rolniczy im. Hugona Kollataja w Krakowie, 31-120 Krakow (PL); MEDGAL Spolka z ograniczona odpowiedzialnoscia, 16-001 Ksiezyno (PL)
(72) Inventor: Aleksiewicz, Roman, 41-100 Siemianowice Slaskie (PL); Ramisz, Grzegorz, 43-400 Cieszyn (PL); Borowska-Skarzynska, Urszula, 15-336 Bialystok (PL); Godlewska, Malgorzata, 18-312 Rutki (PL); Albu, Kateryna, 41-100 Siemianowice Slaskie (PL)
(74) Representative: Hudy, Ludwik

(56) References cited:
- WO-A1-2021/123994
- US-A1- 2019 083 105
- US-A1- 2020 085 452
- US-A1- 2021 282 790

## Description

According to the preamble of claim 1, the present invention relates to a guide of a saw blade, especially relates to a guide of a straight blade of an oscillatory saw for double and triple pelvic osteotomies in dogs.

Canine Hip Dysplasia (CHD) is a common developmental abnormality of the hip joints in dogs. The hip dysplasia is characterized by, among other things, abnormalities in inclination and anteversion of the femoral neck, leading to incongruity of the hip joint components, uneven loading of the joint surface and stretching of the joint capsule.

Among dogs, the disease mainly affects medium-sized dogs weighing from 11 kg to 25 kg, large dogs weighing from 26 kg to 44 kg and very large dogs weighing more than 45 kg, in which the epidemiology of prevalence can be as high as 65%, as in the case of Great Danes. One of the ways to manage dysplasia are corrective pelvic osteotomies which are recognized as effective techniques to prevent the subsequent development of hip joint osteoarthritis.

The first corrective pelvic osteotomy technique, specifically a Triple Pelvic Osteotomy (TPO) in dogs, was introduced by B. Slocum in 1986. The TPO procedure was a three-stage osteotomy of the pelvic bones consisting in excision of a fragment of the pubic bone, incision of an ischium and incision of a diaphysis of the hip bone with closure of the osteotomy using a correction plate. The aim of the procedure was to achieve ventrolateral rotation of the bony element along with an acetabulum of the hip joint. During the surgery, an acetabular segment was rotated to achieve the desired ventral inclination and provide better coverage of a femoral head for a poorly formed acetabular.

As a result of the procedure there was an increase in a bearing surface area within an articular cartilage of the acetabulum which, according to the intention of the authors of the technique, should limit the development of degeneration in the hip joint.

The degree of correction and the choice of osteotomy technique are finally selected during the X-ray or radiographic examination. The introduction of new solutions, such as locked TPO/DPO plates, multiplanar, and the development of new surgical techniques, that are DPO and 2.5PO, have led to a reduction in the invasiveness of the procedure. Despite these changes, no significant tooling, such as a guide for the cutting blade, has been proposed as far. The techniques of TPO, DPO and 2.5PO which differ from each other, are connected by a stage of the procedure consisting in the osteotomy of the hip bone diaphysis. The shortage of a guide forces the operator or surgeon to perform the so-called "freehand cut" of the hip bone diaphysis. As a result of such procedure, intraoperatively, the following may occur: damage to the cortex of the hip bone diaphysis, non-orthogonal osteotomy planes, soft tissue damage. Non-orthogonality of the osteotomy surface in the postoperative period becomes a cause of implant loosening, excessive coverage of the femoral head by the acetabular cap, and/or delayed healing of the hip bone and ischial bone osteotomies. The postoperative complication rate ranges from 35% to 70% of cases.

The TPO technique described by B. Slocum provides essential rules for its implementation. The first step of the procedure is determination of an auxiliary line which is a long axis of the pelvis which is led from the ischial tuberosity through the highest point of the acetabular labrum cephalad and which divides the pelvis in a longitudinal axis. The second step of the procedure is determination of an osteotomy line which is led from the point determined by the dorsocaudal iliac spine of the ilium at an angle of 90° to the previously determined auxiliary line which is the long axis of the pelvis.

Determining the osteotomy line at the angle of 90° in relation to the long axis of the pelvis guarantees that the bone screws stabilizing the TPO/DPO plate in the cranial fraction of the osteotomy, passing through a wing of the ilium will be inserted at the level of an auricular surface into a diaphysis of sacrum. This is a key stage of osteosynthesis, guaranteeing its stability and strength. So far, the technique of determining the osteotomy line has not been optimized by any procedure or tool and the iliac shaft or diaphysis osteotomy has been performed using the so-called "freehand cut".

Publication US 2019/083105 A1 of the patent application description titled "Bone cutting guide system for osteochondral transplantation" presents a bone cutting guide for preparing both donor and recipient bone, including one or more articular referencing platforms contoured to a bony surface to be prepared, fixation structure to secure the articular referencing platforms on an articular surface, cutting slots spaced apart from the articular referencing platforms at predetermined distances configured to allow the passage of a saw blade in such a way to remove a bone segment either from an allograft donor or from a graft recipient.

In turn, publication US 2020/085452 A1 of the patent application description titled "Patient-specific template for total ankle replacement" discloses an alignment guide for total ankle replacement surgery and a method of creating and using the alignment guide. The alignment guide has a surface that interfaces with a corresponding surface of a superior aspect a talus of a patient, and as second surface portion that interfaces with a corresponding distal surface of a section of a tibia of the patient. When the alignment guide is in position, it maintains the ankle of the patient in a preselected position.

Publication US 2021/282790 A1 of the patent application description titled "Patient-specific ankle guide systems and methods" teaches devices for assisting in performing an ankle arthroplasty on a non-resected bone surface of a tibia and/or a talus. The devices have patient-specific mating surfaces configured to engage the non-resected bone surface in a single relative position relative to the non-resected bone surface. The patient-specific nature of the mating surface portion may be generated in the devices prior to the devices being brought into contact with the bone. The devices may include various cutting guides and holes for receiving fasteners to fasten the devices to the bone and various features to enhance the stability and/or surface area contact between the devices and the bones.

Furthermore, publication WO 2021/123994 A1 of the international patent application description titled "Limb and joint sparing in mammals using patient-specific surgical guides and implant with textured muscle attachment zones" shows patient-specific surgical guides used for replacing an excised of proximal humerus. The cutting guide's shape is primarily influenced by the shape of the bone to excise and the cutting guide length depends on the resection margin established by the surgeon to prevent tumor recurrence.

The above described guides are suitable for using the guides at cutting of bones of dedicated pets or humans The aim of the invention is to create a tool that would enable precise determination of an osteotomy line and perform a cutting of a hip bone in an optimized manner, ensuring an orthogonality of osteotomy planes.

The aim is achieved according to the invention by means of a guide of a cutting tool blade or a saw, having the features of claim 1. Advantageous embodiments of the invention are defined in the respective dependent claims.

According to the invention, a guide of a saw blade comprises a guiding element whose body has a shape of a solid fitting into a cuboid and which has a longitudinal through hole or a slit for guiding the cutting tool blade used for the procedure having a width not greater than 3,0 mm and a length b smaller than a longitudinal dimension, i.e. the longest dimension of the body of the guiding element that is situated perpendicular to a bottom wall or surface whereby the guiding element has a set of at least three through mounting holes with a diameter not greater than 3,0 mm whose outlets are located within the bottom wall or surface of the guiding element, two of which are situated on one side of the longitudinal through hole or slit and at least one is situated on an opposite side of a longitudinal axis of symmetry of the through hole or slit in relation to a side on which the two through mounting holes are located, whereas orthogonal projections of the outlets of the most distanced through mounting holes from each other and situated on one side of the longitudinal through hole or slit on the longitudinal axis of symmetry of the through hole or slit are located between opposite walls of the through hole or slit wherein the guide of the saw blade has a slider and in the body of the guiding element there is a transverse through hole perpendicular to the longitudinal axis of symmetry of the longitudinal through hole with a cross-sectional shape and dimensions corresponding to a shape and dimensions of a cross-sectional profile of the slider and the slider is movably positioned within the transverse through hole and has a stabilizing hole nearby its free end and the guide of the saw blade has a locking screw and in the body of the guiding element there is a threaded hole that runs from an upper wall or surface of the guiding element to at least the transverse through hole and in which a locking screw is screwed in.

In one preferred embodiment the guide of saw blade is suitable for a straight blade of the saw for double and triple pelvic osteotomies in dogs.

Preferably, the bottom wall or surface of the guiding element is flat and the through hole is perpendicular to the flat bottom wall or surface of the guiding element.

According to one preferred embodiment, a particularly advantageous use of the guide according to the invention can be seen in that the body of the guiding element is a board having a shape of rectangular parallelepiped or cuboid with a base that is a rectangle with rounded corners.

There can be recesses or indentations in a side walls of the body of the guiding element.

According to another structural variant of the invention, it is advantageous that the guide has a cylindrical insert with a longitudinal through hole of a width not exceeding 3,0 mm whose outer contour of a cross-section corresponds in shape and dimensions to a longitudinal hole made in the body of the guiding element enlarged in all transverse directions by a thickness of the walls of the cylindrical insert compared to the longitudinal through hole.

The guiding element can be formed as a board with a thickness ranging from 15,0 mm to 20,0 mm, a width ranging from 20,0 mm to 30,0 mm and a length ranging from 35,0 mm to 45,0 mm.

According to a further development of the invention, this is particularly advantageous that the set of mounting holes has an even number of mounting holes forming two sub-sets of mounting holes with an equal number of mounting holes whereas the sub-sets of mounting holes are located on opposite sides of the longitudinal through hole or slit and distances of holes from edges of the longitudinal through hole or slit are the same.

The main aspect of the design of the straight oscillating saw blade guide are the guiding element and the slider of the guide. The combination of the slider and the guiding element allows for the determination of the osteotomy line at an angle of 90° relative to the long axis of the pelvis. The effect of using the guide is the ability to cut the hip bone of an animal, especially a dog, in a precisely defined place.

The subject of the invention has been shown in the attached drawing, where:
Fig. 1 shows an exemplary embodiment of a guide of a straight blade of an oscillatory saw in an axonometric view;
Fig. 2 shows a top view of the guide of the straight blade of the oscillatory saw from Fig. 1;
Fig. 3 shows a cross-sectional view of the guide of the straight blade of the oscillatory saw from Fig. 1;
Fig. 4 shows a longitudinal sectional view of the guide of the straight blade of the oscillatory saw from Fig. 1;
Fig. 5 shows a bottom view of the guide of the straight blade of the oscillatory saw from Fig. 1;
Fig. 6 shows another embodiment of a guide of a straight blade of an oscillatory saw in an axonometric view;
Fig. 7 shows a top view of the guide of the straight blade of the oscillatory saw from Fig. 6;
Fig. 8 shows a plan view of yet another guide of a straight blade of an oscillatory saw; and
Figs. 9 and 10 show a guide of a straight blade of an oscillatory saw attached to a hip bone or ilium prior to surgery, for example, an iliac shaft or diaphysis osteotomy performed as part of a corrective pelvic osteotomy in dogs.

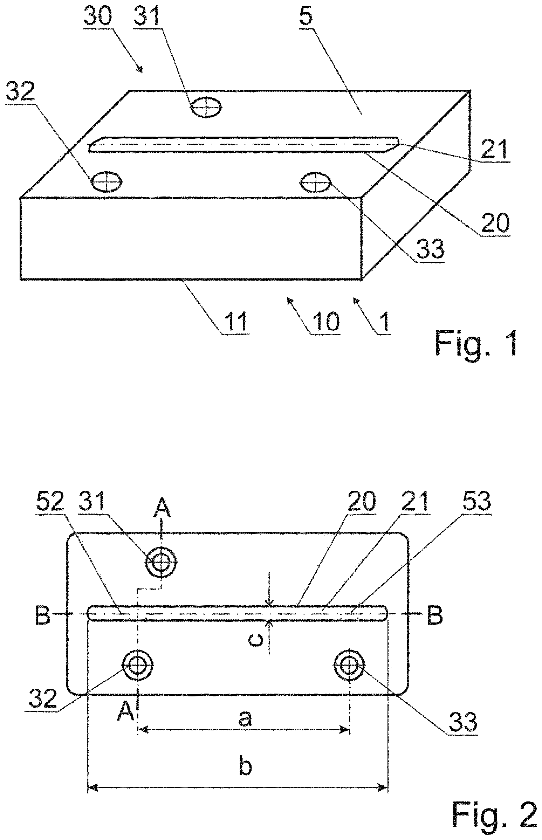

A guide 1 of a saw blade 3 or other cutting tool in the embodiment shown in Figs. 1-5 comprises a guiding element 10 whose body 5 has a shape of a solid fitting into a cuboid. In one embodiment the guiding element 10 has a longitudinal through hole or a slit 20 having a width c adapted to the thickness of the saw blade or other cutting tool, in particular not greater than 3,0 mm and a length b smaller than a longitudinal dimension of the body 5 of the guiding element 10. In particular, the longitudinal through hole or slit 20 is located in the middle of the solid fitting into a cuboid. Preferably, the length b of the hole is selected on the basis of X-ray images of the bones, such that said length is greater than the width of the bone at the intended osteotomy site. The length of the longitudinal through hole or slit can be adjusted according to the anticipated surgical conditions. The longitudinal through hole or slit 20 in particular is perpendicular to the bottom wall of the guiding element 10 and in the embodiment shown in Figs. 1-5 is formed directly in the material of the longitudinal body 5. In one embodiment the bottom wall or surface 11 is flat.

In one of the embodiments the blade of the cutting tool is the straight blade of the saw.

In addition to the longitudinal through hole 20, the guide 1 has a set 30 of at least three through mounting holes 31, 32, 33 made in the body 5 of the guiding element 10 with a diameter not greater than 3,0 mm whose outlets 41, 42, 43 are located within the bottom wall or surface 11, two of which are situated on one side of the longitudinal through hole or slit 20 and one is situated on an opposite side of a longitudinal axis 21 of symmetry of the through hole or slit 20 in relation to a side on which the two through mounting holes are located. Orthogonal projections 52, 53 of the outlets 42, 43 of the most distanced through mounting holes 32, 33 from each other and situated on one side of the longitudinal through hole or slit 20 on the longitudinal axis 21 of symmetry of the through hole or slit 20 are located between opposite walls of the through hole or slit 20. This means that the distance a between the through holes 32, 33, in particular between their centers, furthest apart and located on one side of the longitudinal through hole 20, is less than the length b of the longitudinal through hole 20 and the through mounting holes 32, 33 as viewed from a side, are located against the background of the longitudinal through hole 20. The diameter of the mounting holes is not greater than 2,5 mm, in particular it is 1,6 mm.

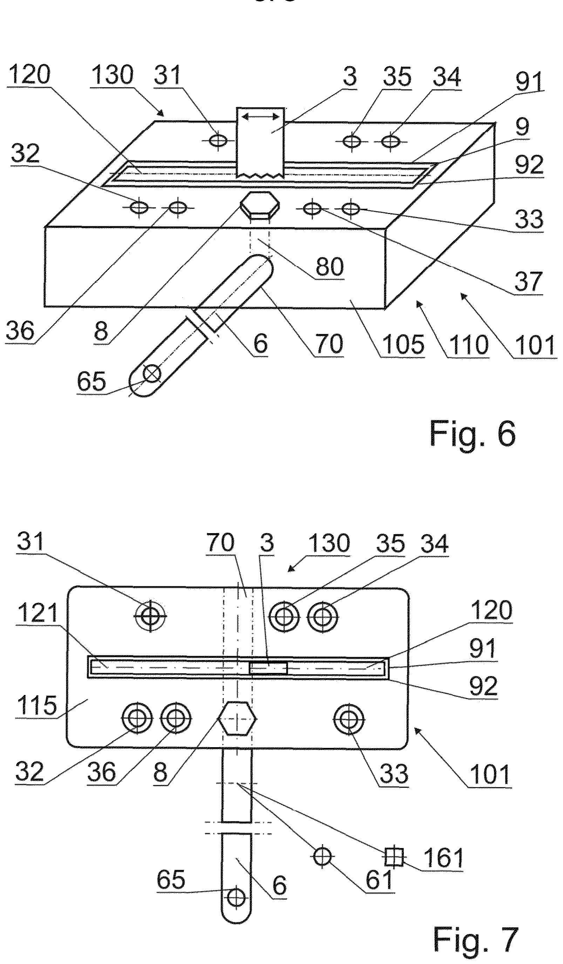

In the embodiment shown in Figs. 6 and 7, a guide 101 of a straight blade 3, shown in fragments, for example, of an oscillatory saw, comprises a guiding element 110 whose body 105 has a shape of a solid fitting into a cuboid. The guiding element 110, in particular in the central part, has a longitudinal through hole or a slit 120 having a width c not greater than 3,0 mm and a length b smaller than a longitudinal dimension of the body 105 of the guiding element 110, except that compared to the embodiment shown in Figs. 1-5, the longitudinal through hole 120 which in this embodiment is a hole 91 and which is perpendicular to a flat bottom wall or surface of the guiding element 110 and which is made in a metal cylindrical insert 9 embedded in a hole 92 made in the longitudinal body 105 and enlarged, in relation to the longitudinal through hole 20 shown in Figs. 1-5, by the thickness of walls of the cylindrical insert 9. The metal cylindrical insert 9 in one embodiment has a rectangular outer cross-sectional contour. The guiding element is made of metal, in particular of steel or cast steel, and a slider is made of steel. In other embodiments, the guiding element is made of plastic, and the cylindrical insert 9, whose external shape and dimensions correspond to a shape of a hole made in the guiding element, is made of metal, in particular of steel or cast steel or other abrasion resistant material, which reduces the friction of the oscillatory saw against inner walls of the longitudinal through hole 120.

As in the embodiment shown in Figs. 1-3 and 5, the guide 101 has a set 130 of through mounting holes, in this case seven through mounting holes 31, 32, 33, 34, 35, 36, 37 made in the body 105 of the guiding element 110 with a diameter not greater than 3,0 mm, whose outlets are located within the bottom wall or surface. The holes 32, 33, 36, 37 are situated on one side of the longitudinal through hole or slit 120, and the holes 31, 34, 35 are situated on an opposite side of a longitudinal axis 121 of symmetry of the longitudinal through hole or slit 120 in relation to a side on which the through mounting holes 32, 33, 36, 37 are located. The mounting holes in both embodiments are used to embed Kirschner nails in them when attaching the plate to the hip bone or ilium bone which stabilize the plate on the hip bone after installation which ensures the stability of the guide, enabling orthogonal cutting of the hip bone during osteotomy. The diameter of the mounting holes is not greater than 2,5 mm, in particular it is 1,6 mm.

The guide 101 of the straight blade 3 of the oscillatory saw in the embodiment shown in Figs. 6 and 7 furthermore has a transverse through hole 70 and a slider 6 with a stabilizing hole 65 with a diameter not greater than 1,8 mm, in particular 1,6 mm, situated at a free end of the slider 6. The transverse through hole 70, with a diameter or a side of 5,0 mm in one embodiment or 6,0 mm in another embodiment, is formed in the body 105 of the guiding element 110 and is located perpendicular to the longitudinal axis 121 of symmetry of the longitudinal through hole or slit 120. The slider 6 is no longer than 80,0 mm, in particular 57,0 mm in one embodiment or 68,0 mm in another embodiment. The transverse through hole 70 acts as a guide for the slider 6 which is movably positioned within the transverse through hole 70, for example with a sliding fit or with a loose fit. The cross section of the hole has a shape whose dimensions correspond to a shape and dimensions of a cross section 61 of the slider 6, for example a circular shape 61 or a square shape 161.

The guide 101 of the straight blade 3 of the oscillatory saw in the embodiment shown in Figs. 6 and 7, additionally has in the body 105 of the guiding element 110 a threaded hole 80 with a diameter not greater than 6,0 mm that runs from an upper wall or surface 115 of the guiding element 110 to at least the transverse through hole 70 and in which a locking screw 8 is screwed in which when screwed into the threaded hole 80, presses the slider 6 against the wall of the longitudinal through hole 70 and initially stabilizes the position of the guide 101 relative to the hip bone.

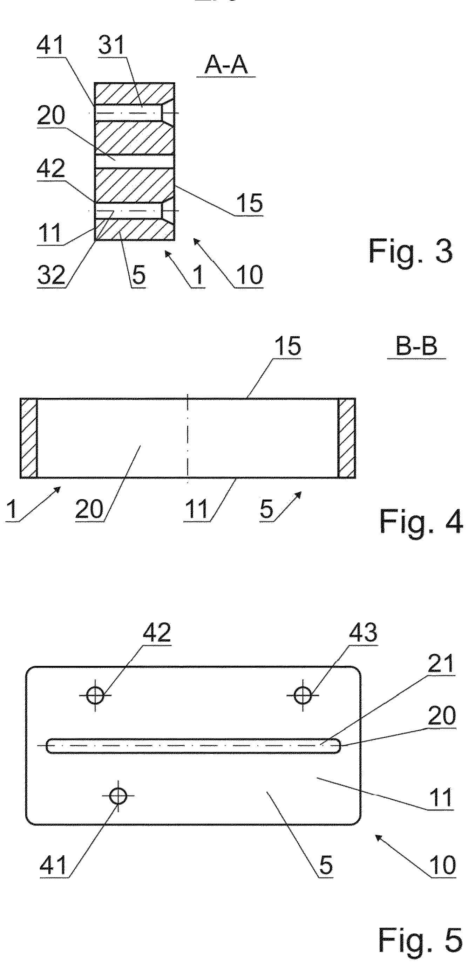

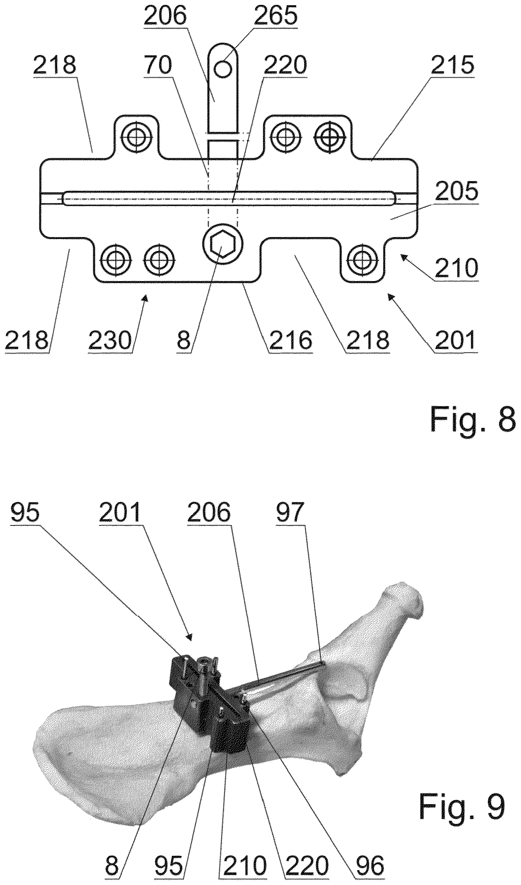

Yet another embodiment of the guide 201 according to the invention is shown in top view in Fig. 8. Compared to the previously described guides, the guide 201 has recesses or indentations 218 in long side walls 216 of a body 205 of a guiding element 210. In one embodiment, the recesses or indentations run perpendicularly to an upper wall or surface 215 of the body 205 of the guiding element 210 whose body 205 has the shape of a solid fitting into a cuboid. The guiding element 210, in particular in the central part, has a longitudinal through hole or slit 220 having a width not greater than 3,0 mm and a length smaller than a longitudinal dimension of the body 205 of the guiding element 210. The longitudinal through hole or slit 220 is perpendicular to the flat bottom wall or surface of the guiding element 210. Similar to the embodiment shown in Fig. 5, the guide 201 has a set 230 of through mounting holes, and similar to the embodiment shown in Figs. 6 and 7, the guide 201 of a straight blade of an oscillatory saw of Figs. 8, 9 and 10 has the transverse through hole 70 and a slider 206 with a stabilizing hole 265 located nearby its free end. The transverse through hole 70 is made in the body 205 of the guiding element 210 and is perpendicular to the longitudinal axis of symmetry of the longitudinal through hole or slit 220. The guide 201 of the straight blade of the oscillatory saw in the embodiment shown in Fig. 8 has yet in the body 205 of the guiding element 210 the threaded hole 80, shown in Fig. 6, with the diameter not greater than 6,0 mm that runs perpendicularly from the upper wall or surface 215 of the guiding element 210 to at least the transverse through hole 70 and in which the locking screw 8 is screwed in which has a similar function as in the examples previously described.

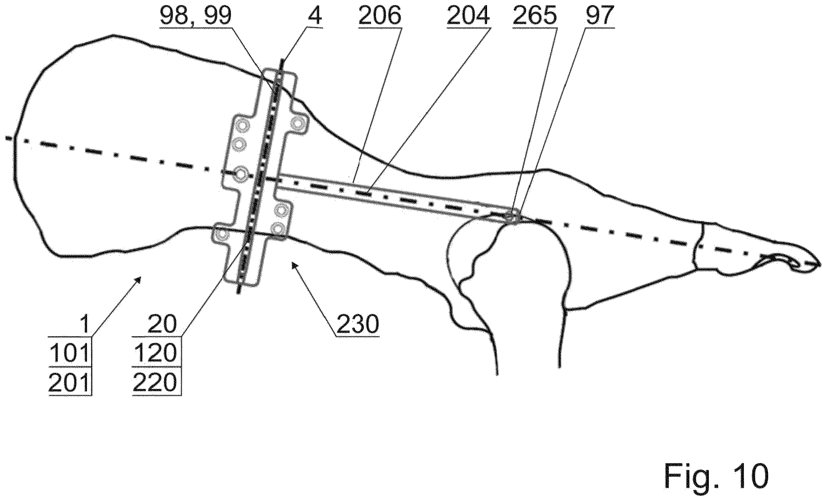

Figs. 9 and 10 show the guide 201 attached to the hip bone or ilium bone prior to the surgery, for example an iliac shaft or diaphysis osteotomy performed as part of a corrective pelvic osteotomy in dogs. Although the following description will refer to the elements of the guide 201 shown in Fig. 8, however, they may as well be the elements of the guides 1 and 101 and variants thereof.

In one embodiment, the set of mounting through holes has an even number of mounting holes forming two sub-sets of mounting holes with an equal number of mounting holes, whereas the sub-sets of mounting holes are located on opposite sides of the longitudinal through hole or slit 20, 120, 220, and distances of holes from edges of the longitudinal through hole or slit 20, 120, 220 are the same.

The dimensions of the guiding element are usually adapted to the size of the hip bone of the operated animal. In one embodiment, the guiding element is a board with a thickness 15,0 mm, in another embodiment 20,0 mm, a width 20,0 mm, in another embodiment 25,0 mm, and in yet another embodiment 30,0 mm, and a length 35,0 mm, and in another embodiment 40,0 mm, and in yet another embodiment 45,0 mm. Given dimensions are also the dimensions of the guiding element shown in Figs. 1, 6 and 8. In all embodiments, the length b of the longitudinal through hole is at least 5,0 mm smaller than the length of the guiding element and the distance a between the extreme or furthest apart mounting holes is at least 8,0 mm smaller.

The guiding element is made of metal, in particular of steel or cast steel, and the slider is made of steel. In other embodiments, the guiding element is made of plastic and the insert is made of steel.

Prior to the above-mentioned procedure, according to the treatment techniques known from the state of the art, the long axis A of the pelvis is determined, previously called the auxiliary line which is cephalad led from the ischial tuberosity through the highest point of the acetabular labrum and which divides the pelvis in the longitudinal axis. The long axis A of the pelvis is marked in Fig. 10 with the numeral 204. Next, the short axis B of the pelvis is lowered from the dorsocaudal iliac spine 98, marked in Fig. 10 with the numeral 4, which runs in relation to the long axis of the pelvis at an angle of 90°. The course of the short axis B of the pelvis is the osteotomy line, the determination of which begins with the placement of Kirschner nails ϕ 1.2 mm, with a length of 20 mm, at the height of the dorsocaudal iliac spine of the ilium. In the next step, a Kirschner nail ϕ 1,5 mm, 20 mm long, is placed above the center of the acetabular labrum. The through hole or a slit 20, 120, 220 of the guide 1, 101, 201 is placed on the Kirschner nail 99 located on the iliac spine. Then, the stabilizing hole 265 of the slider 6 of the guide 101, 201 is placed on the Kirschner nail 97 located at the height of the center of the acetabular labrum. In this arrangement of the guide with the slider the movement of the slider 6 is blocked by means of the locking screw 8 shown in Fig. 9. Stabilization of the slider with the guiding element takes place in their mutual juxtaposition at an angle of 90°. In the next step, the guide is stabilized on the iliac bone shaft or diaphysis by placing the Kirschner nails ϕ 1,50 mm with a length of 20 mm in the mounting holes. In the next step, the guiding element for osteotomy is prepared by removing the Kirschner nails 97, 99 placed on the dorsocaudal iliac spine of the ilium and at the point above the acetabular labrum. After unlocking the locking screw and extending the guide slider, the remaining Kirschner nails ϕ 1,50 mm are shortened to 0,8 mm above the guide surface using wire pliers so that they do not hinder the osteotomy which is performed in % of the thickness of the iliac shaft or diaphysis using the straight blade of the oscillatory saw of 1,00 mm thick. The final step involves taking out the guiding element from the Kirschner nails positioned within the holes of the set of 230 mounting holes shown in Fig. 8 and their removal.

## Claims

1. A guide (1, 101, 201) of a saw blade (3) for double and triple pelvic osteotomies in dogs comprising a guiding element (10, 110, 210)
whose body (5, 105, 205) has a shape of a solid fitting into a cuboid and which has a longitudinal through hole or a slit (20, 120, 220) having a width c not greater than 3,0 mm and a length b smaller than a longitudinal dimension of the body (5, 105, 205) of the guiding element (10, 110, 210), whereby the through hole or slit (20, 120, 220) is perpendicular to a bottom wall or surface (11, 111, 211) of the guiding element (10, 110, 210)
and which has a set (30) of at least three through mounting holes (31, 32, 33) with a diameter not greater than 3,0 mm whose outlets (41, 42, 43) are located within the bottom wall or surface (11) of the guiding element (10, 110, 210), two of which are situated on one side of the longitudinal through hole or slit (20, 120) and at least one is situated on an opposite side of a longitudinal axis (21) of symmetry of the through hole or slit (20, 120) in relation to a side on which the two through mounting holes are located, whereas orthogonal projections (52, 53) of the outlets (42, 43) of most distanced through mounting holes (32, 33) from each other and situated on one side of the longitudinal through hole or slit (20) on the longitudinal axis (21) of symmetry of the through hole or slit (20) are located between opposite walls of the through hole or slit (20)
**characterised in that** the guide has a slider (6, 206) and in the body (105, 205) of the guiding element (110, 210) there is a transverse through hole (70) perpendicular to the longitudinal axis (121) of symmetry of the longitudinal through hole (120, 220) with a cross-sectional shape and dimensions corresponding to a shape and dimensions of a cross-sectional profile of the slider (6, 206) and the slider (6, 206) is movably positioned within the transverse through hole (70) and has a stabilizing hole (65, 265) nearby its free end and
has a locking screw (8) and in the body (105, 205) of the guiding element (110, 210) there is a threaded hole (80) that runs from an upper wall or surface (115, 215) of the guiding element (110, 210) to at least the transverse through hole (70) and in which a locking screw (8) is screwed in.

2. The guide (1, 101, 201) of the saw blade (3) according to the claim 1, **characterized in that** the guide of the saw blade is suitable for a straight blade (3) of the saw for double and triple pelvic osteotomies in dogs.

3. The guide (101, 201) of the saw blade (3) according to claim 1 or 2, **characterized in that** the bottom wall or surface (11) of the guiding element (110, 210) is flat and the through hole (120, 220) is perpendicular to the flat bottom wall or surface (11) of the guiding element (110, 210).

4. The guide (1, 101, 201) for the saw blade (3) according to claim 1 or 2 or 3, **characterized in that** the body (5) of the guiding element (10) is a board having a shape of rectangular parallelepiped or cuboid with a base that is a rectangle with rounded corners.

5. The guide (201) for the saw blade (3) according to claim 1 or 2 or 3 or 4, **characterized in that** there are recesses or indentations (218) in side walls (216) of the body (205) of the guiding element (210).

6. The guide (101) for the saw blade (3) according to claim 1 or 2 or 3 or 4 or 5, **characterized in that** the guide has a cylindrical insert (9) with a longitudinal through hole (120) of a width not exceeding 3,0 mm whose outer contour of a cross-section (92) corresponds in shape and dimensions to a longitudinal hole (91) made in the body (105) of the guiding element (110) enlarged in all transverse directions by a thickness of the walls of the cylindrical insert (9) compared to the longitudinal through hole (120).

7. The guide (1, 101, 201) for the saw blade (3) according to claim 1 or 2 or 3 or 4 or 5 or 6, **characterized in that** the guiding element (210) is a board with a thickness ranging from 15,0 mm to 20,0 mm, a width ranging from 20,0 mm to 30,0 mm, and a length ranging from 35,0 mm to 45,0 mm.

8. The guide (1, 101, 201) for the saw blade (3) according to claim 1 or 2 or 3 or 4 or 5 or 6 or 7, **characterized in that** the set (30) of mounting holes has an even number of mounting holes forming two sub-sets of mounting holes with an equal number of mounting holes whereas the sub-sets of mounting holes are located on opposite sides of the longitudinal through hole or slit (20, 120, 220) and distances of holes from edges of the longitudinal through hole or slit (20, 120, 220) are the same.

## Patentansprüche

1. Eine Führung (1, 101, 201) eines Sägeblattes (3) für Doppel- und Dreifachbeckenosteotomien bei Hunden, umfassend ein Führungselement (10, 110, 210),
dessen Körper (5, 105, 205) eine Form eines Festmaterials aufweist, das in einen Quader passt, und das ein längliches Durchgangsloch oder einen Schlitz (20, 120, 220) mit einer Breite c von nicht mehr als 3,0 mm und einer Länge b kleiner als eine Längsabmessung des Körpers (5, 105, 205) des Führungselements (10, 110, 210) aufweist, wobei das Durchgangsloch oder der Schlitz (20, 120, 220) senkrecht zu einer unteren Wand oder Oberfläche (11, 111, 211) des Führungselements (10, 110, 210) ist,
und das einen Satz (30) von zumindest drei Durchgangsmontagelöchern (31, 32, 33) mit einem Durchmesser von nicht mehr als 3,0 mm aufweist, deren Auslässe (41, 42, 43) sich innerhalb der unteren Wand oder Oberfläche (11) des Führungselements (10, 110, 210) befinden, von denen zwei auf einer Seite des länglichen Durchgangslochs oder Schlitzes (20, 120) platziert sind und zumindest einer auf einer gegenüberliegenden Seite einer Längssymmetrieachse (21) des Durchgangslochs oder Schlitzes (20, 120) in Bezug auf eine Seite platziert ist, auf der sich die zwei Durchgangsmontagelöcher befinden, während sich orthogonale Projektionen (52, 53) der Auslässe (42, 43) von am weitesten voneinander beabstandeten Durchgangsmontagelöchern (32, 33) und die auf einer Seite des länglichen Durchgangslochs oder Schlitzes (20) auf der Längssymmetrieachse (21) des Durchgangslochs oder Schlitzes (20) platziert sind zwischen gegenüberliegenden Wänden des Durchgangslochs oder Schlitzes (20) befinden,
**dadurch gekennzeichnet, dass** die Führung einen Schieber (6, 206) aufweist und es in dem Körper (105, 205) des Führungselements (110, 210) ein Querdurchgangsloch (70) senkrecht zu der Längssymmetrieachse (121) des länglichen Durchgangslochs (120, 220) mit einer Querschnittsform und Abmessungen gibt, die einer Form und Abmessungen eines Querschnittsprofils des Schiebers (6, 206) entsprechen, und der Schieber (6, 206) bewegbar innerhalb des Querdurchgangslochs (70) positioniert ist und ein Stabilisierungsloch (65, 265) nahe seinem freien Ende aufweist und eine Feststellschraube (8) aufweist und es in dem Körper (105, 205) des Führungselements (110, 210) ein Gewindeloch (80) gibt, das von einer oberen Wand oder Oberfläche (115, 215) des Führungselements (110, 210) zu zumindest dem Querdurchgangsloch (70) verläuft und in das eine Feststellschraube (8) eingeschraubt ist.

2. Die Führung (1, 101, 201) des Sägeblattes (3) nach Anspruch 1, **dadurch gekennzeichnet, dass** die Führung des Sägeblattes für ein gerades Blatt (3) der Säge für Doppel- und Dreifachbeckenosteotomien bei Hunden geeignet ist.

3. Die Führung (101, 201) des Sägeblattes (3) nach Anspruch 1 oder 2, **dadurch gekennzeichnet, dass** die untere Wand oder Oberfläche (11) des Führungselements (110, 210) flach ist und das Durchgangsloch (120, 220) senkrecht zu der flachen unteren Wand oder Oberfläche (11) des Führungselements (110, 210) ist.

4. Die Führung (1, 101, 201) für das Sägeblatt (3) nach Anspruch 1 oder 2 oder 3, **dadurch gekennzeichnet, dass** der Körper (5) des Führungselements (10) eine Platte mit einer Form eines rechteckigen Parallelepipeds oder Quaders mit einer Basis ist, die ein Rechteck mit gerundeten Ecken ist.

5. Die Führung (201) für das Sägeblatt (3) nach Anspruch 1 oder 2 oder 3 oder 4, **dadurch gekennzeichnet, dass** es Aussparungen oder Vertiefungen (218) in Seitenwänden (216) des Körpers (205) des Führungselements (210) gibt.

6. Die Führung (101) für das Sägeblatt (3) nach Anspruch 1 oder 2 oder 3 oder 4 oder 5, **dadurch gekennzeichnet, dass** die Führung einen zylindrischen Einsatz (9) mit einem länglichen Durchgangsloch (120) einer Breite von nicht mehr als 3,0 mm aufweist, dessen Außenkontur eines Querschnittes (92) in Form und Abmessungen einem länglichen Loch (91) entspricht, das in dem Körper (105) des Führungselements (110) ausgebildet ist, das in allen Querrichtungen um eine Dicke der Wände des zylindrischen Einsatzes (9) verglichen mit dem länglichen Durchgangsloch (120) vergrößert ist.

7. Die Führung (1, 101, 201) für das Sägeblatt (3) nach Anspruch 1 oder 2 oder 3 oder 4 oder 5 oder 6, **dadurch gekennzeichnet, dass** das Führungselement (210) eine Platte mit einer Dicke, die von 15,0 mm bis 20,0 mm reicht, einer Breite, die von 20,0 mm bis 30,0 mm reicht, und einer Länge, die von 35,0 mm bis 45,0 mm reicht, ist.

8. Die Führung (1, 101, 201) für das Sägeblatt (3) nach Anspruch 1 oder 2 oder 3 oder 4 oder 5 oder 6 oder 7, **dadurch gekennzeichnet, dass** der Satz (30) von Montagelöchern eine gerade Anzahl an Montagelöchern aufweist, die zwei Untersätze von Montagelöchern mit einer gleichen Anzahl an Montagelöchern bilden, während sich die Untersätze von Montagelöchern auf gegenüberliegenden Seiten des länglichen Durchgangslochs oder Schlitzes (20, 120, 220) befinden und Abstände von Löchern von Kanten des länglichen Durchgangslochs oder Schlitzes (20, 120, 220) gleich sind.

## Revendications

1. Guide (1, 101, 201) d'une lame de scie (3) pour ostéotomies pelviennes doubles et triples chez les chiens, comprenant un élément de guidage (10, 110, 210) dont le corps (5, 105, 205) comporte la forme d'un solide s'ajustant dans un cuboïde et qui comporte un trou traversant longitudinal ou une fente (20, 120, 220) comportant une largeur c non supérieure à 3,0 mm et une longueur b inférieure à une dimension longitudinale du corps (5, 105, 205) de l'élément de guidage (10, 110, 210), moyennant quoi le trou traversant ou la fente (20, 120, 220) est perpendiculaire à une paroi ou surface inférieure (11, 111, 211) de l'élément de guidage (10, 110, 210) et qui comporte un ensemble (30) d'au moins trois trous de montage traversants (31, 32, 33) d'un diamètre non supérieur à 3,0 mm dont les sorties (41, 42, 43) sont situées à l'intérieur de la paroi ou surface inférieure (11) de l'élément de guidage (10, 110, 210), dont deux sont placés sur un côté du trou traversant ou de la fente longitudinale (20, 120) et au moins un est situé sur un côté opposé d'un axe longitudinal (21) de symétrie du trou traversant ou de la fente (20, 120) par rapport à un côté sur lequel se trouvent les deux trous de montage traversants, tandis que des projections orthogonales (52, 53) des sorties (42, 43) des trous de montage traversants (32, 33) les plus éloignés les uns des autres et placés sur un côté du trou traversant ou de la fente longitudinale (20) sur l'axe longitudinal (21) de symétrie du trou traversant ou de la fente (20) sont situées entre des parois opposées du trou traversant ou de la fente (20) **caractérisé en ce que** le guide comporte un coulisseau (6, 206) et dans le corps (105, 205) de l'élément de guidage (110, 210) se trouve un trou traversant transversal (70) perpendiculaire à l'axe longitudinal (121) de symétrie du trou traversant longitudinal (120, 220) avec une forme de section transversale et des dimensions correspondant à une forme et des dimensions d'un profil de section transversale du coulisseau (6, 206) et le coulisseau (6, 206) est positionné de manière mobile à l'intérieur du trou traversant transversal (70) et comporte un trou de stabilisation (65, 265) à proximité de son extrémité libre et comporte une vis de blocage (8) et dans le corps (105, 205) de l'élément de guidage (110, 210) se trouve un trou fileté (80) qui s'étend à partir d'une paroi ou surface supérieure (115, 215) de l'élément de guidage (110, 210) jusqu'au moins le trou traversant transversal (70) et dans lequel une vis de blocage (8) est vissée.

2. Guide (1, 101, 201) de la lame de scie (3) selon la revendication 1, **caractérisé en ce que** le guide de la lame de scie convient à une lame droite (3) de la scie pour ostéotomies pelviennes doubles et triples chez les chiens.

3. Guide (101, 201) de la lame de scie (3) selon la revendication 1 ou 2, **caractérisé en ce que** la paroi ou surface inférieure (11) de l'élément de guidage (110, 210) est plate et le trou traversant (120, 220) est perpendiculaire à la paroi ou surface inférieure plate (11) de l'élément de guidage (110, 210).

4. Guide (1, 101, 201) pour la lame de scie (3) selon la revendication 1 ou 2 ou 3, **caractérisé en ce que** le corps (5) de l'élément de guidage (10) est un panneau présentant une forme de parallélépipède rectangle ou de cuboïde rectangle avec une base qui est un rectangle à coins arrondis.

5. Guide (201) pour la lame de scie (3) selon la revendication 1 ou 2 ou 3 ou 4, **caractérisé en ce que** des évidements ou des indentations (218) se trouvent dans les parois latérales (216) du corps (205) de l'élément de guidage (210).

6. Guide (101) pour la lame de scie (3) selon la revendication 1 ou 2 ou 3 ou 4 ou 5, **caractérisé en ce que** le guide comporte un insert cylindrique (9) avec un trou traversant longitudinal (120) d'une largeur ne dépassant pas 3,0 mm dont le contour externe d'une section transversale (92) correspond en forme et en dimensions à un trou longitudinal (91) réalisé dans le corps (105) de l'élément de guidage (110) agrandi dans toutes les directions transversales d'une épaisseur des parois de l'insert cylindrique (9) par rapport au trou traversant longitudinal (120).

7. Guide (1, 101, 201) pour la lame de scie (3) selon la revendication 1 ou 2 ou 3 ou 4 ou 5 ou 6, **caractérisé en ce que** l'élément de guidage (210) est un panneau possédant une épaisseur allant de 15,0 mm à 20,0 mm, une largeur allant de 20,0 mm à 30,0 mm et une longueur allant de 35,0 mm à 45,0 mm.

8. Guide (1, 101, 201) pour la lame de scie (3) selon la revendication 1 ou 2 ou 3 ou 4 ou 5 ou 6 ou 7, **caractérisé en ce que** l'ensemble (30) de trous de montage comporte un nombre pair de trous de montage formant deux sous-ensembles de trous de montage avec un nombre égal de trous de montage tandis que les sous-ensembles de trous de montage sont situés sur des côtés opposés du trou traversant longitudinal ou de la fente (20, 120, 220) et les distances des trous par rapport aux bords du trou traversant longitudinal ou de la fente (20, 120, 220) sont les mêmes.
